# EUROPEAN PATENT APPLICATION

(11) **EP 3 330 674 A1**
(43) Date of publication of application: **06.06.2018**
(21) Application number: 15889029.3
(22) Date of filing: 24.11.2015
(51) Int. Cl.: G01C 22/00

(54) **METHOD AND APPARATUS FOR REALIZING STEP COUNTING**

(30) Priority: 08.09.2015 CN 201510567832
(71) Applicant: ZTE Corporation, Shenzhen, Guangdong 518057 (CN); XI'AN UNIVERSITY OF POSTS & TELECOMMUNICATIONS, Xi'an, Shaanxi 710121 (CN)
(72) Inventor: SONG, Hui, Shenzhen Guangdong 518057 (CN); ZHANG, Rong, Shenzhen Guangdong 518057 (CN); WANG, Zhongmin, Shenzhen Guangdong 518057 (CN); LIANG, Chen, Shenzhen Guangdong 518057 (CN); HE, Yan, Shenzhen Guangdong 518057 (CN); HENG, Xia, Shenzhen Guangdong 518057 (CN); FAN, Lin, Shenzhen Guangdong 518057 (CN); WANG, Wenlang, Shenzhen Guangdong 518057 (CN); HE, Feifei, Shenzhen Guangdong 518057 (CN); LU, Chen, Shenzhen Guangdong 518057 (CN); ZHI, Zhou, Shenzhen Guangdong 518057 (CN)
(74) Representative: Taor, Simon Edward William
(86) International application number: PCT/CN2015/095416
(87) International publication number: WO 2016/165333

(57) **Abstract**

A method and apparatus for realizing step counting, comprising: according to acquired step counting data of a pre-set number of steps, correcting a step counting frequency and/or an effective stride value of step counting of a three-axis acceleration sensor (100); and counting the number of steps according to the corrected step counting frequency and/or the corrected effective stride value of step counting of the three-axis acceleration sensor (101). By correcting the step counting frequency and/or the effective stride value of step counting of the three-axis acceleration sensor, the technical solution avoids a step counting error caused by different walking strides of a user and different step counting frequencies of the three-axis acceleration sensor, and improves the accuracy of step counting data; in addition, by correcting an effective step counting frequency, invalid step counting in the step counting data is processed, and the accuracy of the step counting data is further improved.

## Description

### TECHNICAL FIELD

The present invention relates to the field of mobile application, and in particular, to a method and an apparatus for realizing step counting.

### BACKGROUND

A pedometer device may record a number of walking steps daily, so as to monitor user's exercises. It has a significant reference value for keep fit for the user. By utilizing a three-axis acceleration sensor built in a mobile phone to count steps, the user does not need to carry an additional pedometer. Moreover, the step counting data may be uploaded to a server through the mobile phone, and may be processed in statistics and managed, to realize effective analysis of the user's exercise data. Therefore, compared with other pedometer device, it is more convenient to use mobile phone to count steps, and it is more effective for statistical analysis of step counting data.

At present, there are mainly two methods for counting steps. Application No. CN1909699A discloses a method and an apparatus for realizing a step counting function of a mobile terminal. The method includes: measuring a curve of acceleration of a moving object; converting an analog signal into a digital signal and extracting voltage information therefrom; and deriving step counting information from the voltage information. The method uses a fixed calculation method to count steps and does not consider pace and frequency of the walking of the user, and other factors influencing the pace and the frequency. For example, different user may have different paces detected depending on the gait and habit. Depending on the pedometer device being placed in a bag, or is worn on a wrist or a waist and other locations, the detected frequency may also be different. In addition, the three-axis acceleration sensors of different mobile phones may be have different step counting frequencies, a frequency too high or too low will have an impact on the step counting data, and generate an error to a certain degree in the step counting data. Application No. CN102954803 discloses an adaptive processing system and method for step counting based on feature point detection method. The method divides the step counting process into a rule searching phase and a rule determination phase, to realize a function of adaptive step counting with the change of the walking state of the user. The method includes: the step counting process initially entering the rule searching phase by default; each time it is monitored that a walking signal satisfies a decision rule of the rule searching phase, increasing a rule searching counter by one; when the rule searching counter reaches to an upper limit value, adding the upper limit value to a total counter, and entering the rule determination phase automatically; and after entering the rule determination phase, each time that it is monitored that a walking signal satisfies a decision rule of the rule determination phase, increasing a rule determination counter and the total counter respectively by one; wherein a decision parameter for the decision rule in each phase may be adaptively adjusted according to the change of the walking state of the user. When it is monitored that the walking signal satisfies the requirement for searching a rule again, the process enters the rule searching phase again. The step counting process includes calculation of combining acceleration data of the three axes, analog-digital conversion and filtering, feature point detection and rule searching decision, counter accumulation, rule determination decision and so on, which requires a large amount of calculation and is complex to implement. It is not convenient for implementation on a terminal device with weak calculation capacity and low battery power, and is not convenient for running on a portable terminal.

In summary, utilizing a fixed calculation method for step counting detection, it tends to generate an error to a certain degree in the step counting data due to different paces and frequencies of users in the detection process. In addition, different three-axis acceleration sensors of mobile phone having different step counting frequencies may also cause an error in the step counting data. Although a complicated step counting algorithm can improve the accuracy of the step counting data, the calculation amount of the step counting process is huge and complex, which is inconvenient to run on a portable diagnosis device.

### SUMMARY

The following is an overview of the subject matter described in detail herein. This summary is not intended to limit the scope of the claims.

Embodiments of the present invention provide a method and an apparatus for realizing step counting, which can improve the accuracy of step counting with simple algorithm.

An embodiment of the present invention provides a method for realizing step counting, comprising:
calibrating a step counting frequency and/or an effective value of a step counting pace of a three-axis acceleration sensor according to acquired step counting data of a preset number of steps; and
performing step counting according to the calibrated step counting frequency and/or the calibrated effective value of the step counting pace of the three-axis acceleration sensor obtained from the calibration.

Optionally, the method further includes: calibrating an effective step counting frequency to obtain a calibrated effective step counting frequency; and processing ineffective step counting in the step counting data according to the calibrated effective step counting frequency.

Optionally, calibrating the step counting frequency of the three-axis acceleration sensor comprises:
when a step counting value in the step counting data is larger than an actual number of walking steps, reducing the step counting frequency of the three-axis acceleration sensor by a preset step length within a preset frequency range starting from a first frequency, and determining a step counting frequency within the preset frequency range when an error between the step counting value and the actual number of walking steps is the smallest, as the calibrated step counting frequency; and
when the step counting value in the step counting data is smaller than the actual number of walking steps, increasing the step counting frequency of the three-axis acceleration sensor by a preset step length within a preset frequency range starting from a first frequency, and determining a step counting frequency within the preset frequency range when an error between the step counting value and the actual number of walking steps is the smallest, as the calibrated step counting frequency.

Optionally, calibrating the effective value of the step counting pace comprises:
when a step counting value in the step counting data is larger than an actual number of walking steps, increasing the value of the step counting pace by a preset pace adjusting unit within an effective pace range starting from a first pace, and determining a value of the step counting pace within the effective pace range when an error between the step counting value and the actual number of walking steps is the smallest, as the calibrated effective value of the step counting pace; and
when a step counting value in the step counting data is smaller than the actual number of walking steps, decreasing the value of the step counting pace by a preset pace adjusting unit within an effective pace range starting from a first pace, and determining a value of the step counting pace within the effective pace range when an error between the step counting value and the actual number of walking steps is the smallest, as the calibrated effective value of the step counting pace.

Optionally, calibrating an effective step counting frequency comprises:
when a step counting value in the step counting data is larger than the actual number of walking steps, reducing the effective frequency by a preset frequency adjusting unit within an effective step counting frequency range starting from a first effective frequency, and determining an effective frequency within the effective step counting frequency range when an error between the step counting value and the actual number of walking steps is the smallest, as the calibrated effective step counting frequency; and
when a step counting value in the step counting data is smaller than the actual number of walking steps, increasing the effective frequency by a preset frequency adjusting unit within an effective step counting frequency range starting from a first effective frequency, and determining an effective frequency within the effective step counting frequency range when an error between the step counting value and the actual number of walking steps is the smallest, as the calibrated effective step counting frequency.

Optionally, calibrating a step counting frequency and an effective value of a step counting pace of a three-axis acceleration sensor comprises:
after the calibrated step counting frequency is obtained, calibrating the effective value of the step counting pace according to the step counting data based on the calibrated step counting frequency; and
calibrating a step counting frequency, an effective value of a step counting pace and an effective step counting frequency of a three-axis acceleration sensor comprises:
after the calibrated step counting frequency is obtained, calibrating a parameter of the effective value of the step counting pace according to the step counting data based on the calibrated step counting frequency; and calibrating the effective step counting frequency according to the step counting data based on the calibrated effective value of the step counting pace.

Optionally, the method further includes: when the calibration of the step counting frequency does not reduce the error between the step counting value and the actual number of walking steps, taking the first frequency as the calibrated step counting frequency;
when the calibration of the effective value of the step counting pace does not reduce the error between the step counting value and the actual number of walking steps, taking the first pace as the calibrated effective value of the step counting pace; and
when the calibration of the effective step counting frequency does not reduce the error between the step counting value and the actual number of walking steps, taking the first effective frequency as the calibrated effective step counting frequency.

Optionally, calibrating the step counting frequency comprises:
based on machine learning algorithm and migration learning algorithm, taking accelerometer data of the three-axis acceleration sensor of the actual number of walking steps in the step counting data, the first frequency and the step counting value as input, determining a step counting frequency when an error between the step counting value and the actual number of walking steps is the smallest during the calculation within the preset frequency range with the preset step length, as the calibrated step counting frequency;
calibrating the effective value of the step counting pace comprises:
based on machine learning algorithm and migration learning algorithm, taking accelerometer data of the three-axis acceleration sensor of the actual number of walking steps in the step counting data, the first pace and the step counting value as input, determining a value of the step counting pace when an error between the step counting value and the actual number of walking steps is the smallest during the calculation within the effective pace range with the preset pace adjusting unit, as the calibrated effective value of the step counting pace; and
calibrating the effective step counting frequency comprises:
based on machine learning algorithm and migration learning algorithm, taking accelerometer data of the three-axis acceleration sensor of the actual number of walking steps in the step counting data, the first effective frequency and the step counting value as input, determining an effective frequency when an error between the step counting value and the actual number of walking steps is the smallest during the calculation within the effective pace range with the preset frequency adjusting unit, as the calibrated effective step counting frequency.

In another aspect, the present application also provides an apparatus for realizing step counting, comprising: a first calibration unit and a step counting unit.

The first calibration unit is configured to calibrate a step counting frequency and/or an effective value of a step counting pace of a three-axis acceleration sensor according to acquired step counting data of a preset number of steps; and
the step counting unit is configured to perform step counting according to the calibrated step counting frequency and/or the calibrated effective value of the step counting pace of the three-axis acceleration sensor obtained from the calibration.

Optionally, the apparatus further includes: a second calibration unit configured to calibrate an effective step counting frequency to obtain a calibrated effective step counting frequency; and process ineffective step counting in the step counting data from the step counting unit according to the calibrated effective step counting frequency.

Optionally, the first calibration unit is configured to calibrate the step counting frequency of the three-axis acceleration sensor by:
according to acquired step counting data of a preset number of steps,
when a step counting value in the step counting data is larger than an actual number of walking steps, reducing the step counting frequency of the three-axis acceleration sensor by a preset step length within a preset frequency range starting from a first frequency, and determining a step counting frequency within the preset frequency range when an error between the step counting value and the actual number of walking steps is the smallest, as the calibrated step counting frequency; and
when the step counting value in the step counting data is smaller than the actual number of walking steps, increasing the step counting frequency of the three-axis acceleration sensor by a preset step length within a preset frequency range starting from a first frequency, and determining a step counting frequency within the preset frequency range when an error between the step counting value and the actual number of walking steps is the smallest, as the calibrated step counting frequency.
the first calibration unit is configured to calibrate the effective value of the step counting pace by:
   when a step counting value in the step counting data is larger than an actual number of walking steps, increasing the value of the step counting pace by a preset pace adjusting unit within an effective pace range starting from a first pace, and determining a value of the step counting pace within the effective pace range when an error between the step counting value and the actual number of walking steps is the smallest, as the calibrated effective value of the step counting pace; and
   when a step counting value in the step counting data is smaller than the actual number of walking steps, decreasing the value of the step counting pace by a preset pace adjusting unit within an effective pace range starting from a first pace, and determining a value of the step counting pace within the effective pace range when an error between the step counting value and the actual number of walking steps is the smallest, as the calibrated effective value of the step counting pace.

Optionally, the second calibration unit is configured to calibrate an effective step counting frequency by:
according to acquired step counting data of a preset number of steps,
when a step counting value in the step counting data is larger than the actual number of walking steps, reducing the effective frequency by a preset frequency adjusting unit within an effective step counting frequency range starting from a first effective frequency, and determining an effective frequency within the effective step counting frequency range when an error between the step counting value and the actual number of walking steps is the smallest, as the calibrated effective step counting frequency; and
when a step counting value in the step counting data is smaller than the actual number of walking steps, increasing the effective frequency by a preset frequency adjusting unit within an effective step counting frequency range starting from a first effective frequency, and determining an effective frequency within the effective step counting frequency range when an error between the step counting value and the actual number of walking steps is the smallest, as the calibrated effective step counting frequency.

Optionally, the first calibration unit is configured to calibrate a step counting frequency and an effective value of a step counting pace of a three-axis acceleration sensor by:
after calibrating the step counting frequency of the three-axis acceleration sensor to obtain the calibrated step counting frequency according to the acquired step counting data of a preset number of steps, calibrating the effective value of the step counting pace according to the step counting data based on the calibrated step counting frequency; and
the second calibration unit is configured to calibrate a step counting frequency, an effective value of a step counting pace and an effective step counting frequency of a three-axis acceleration sensor by:
after the first calibration unit has completed the calibration of the step counting frequency of the three-axis acceleration sensor and then the calibration of the effective value of the step counting pace sequentially, calibrating the effective step counting frequency according to the step counting data based on the calibrated effective value of the step counting pace.

Optionally, the apparatus further includes: a judgment-assignment unit configured to, when the calibration of the step counting frequency does not reduce the error between the step counting value and the actual number of walking steps, take the first frequency as the calibrated step counting frequency;
when the calibration of the effective value of the step counting pace does not reduce the error between the step counting value and the actual number of walking steps, take the first pace as the calibrated effective value of the step counting pace; and
when the calibration of the effective step counting frequency does not reduce the error between the step counting value and the actual number of walking steps, take the first effective frequency as the calibrated effective step counting frequency.

Optionally, the first calibration unit is configured to calibrate the step counting frequency by:
based on machine learning algorithm and migration learning algorithm, taking accelerometer data of the three-axis acceleration sensor of the actual number of walking steps in the step counting data, the first frequency and the step counting value as input, determining a step counting frequency when an error between the step counting value and the actual number of walking steps is the smallest during the calculation within the preset frequency range with the preset step length, as the calibrated step counting frequency;
the first calibration unit is configured to calibrate the effective value of the step counting pace by:
   based on machine learning algorithm and migration learning algorithm, taking accelerometer data of the three-axis acceleration sensor of the actual number of walking steps in the step counting data, the first pace and the step counting value as input, determining a value of the step counting pace when an error between the step counting value and the actual number of walking steps is the smallest during the calculation within the effective pace range with the preset pace adjusting unit, as the calibrated effective value of the step counting pace; and
the second calibration unit is configured to calibrate the effective step counting frequency comprises:
   based on machine learning algorithm and migration learning algorithm, taking accelerometer data of the three-axis acceleration sensor of the actual number of walking steps in the step counting data, the first effective frequency and the step counting value as input, determining an effective frequency when an error between the step counting value and the actual number of walking steps is the smallest during the calculation within the effective step counting frequency range with the preset frequency adjusting unit, as the calibrated effective step counting frequency.

An embodiment of the present invention also provide a computer storage medium having stored therein computer-executable instructions for performing the above method.

Compared with the related art, the technical solution of the present application includes: calibrating a step counting frequency and/or an effective value of a step counting pace of a three-axis acceleration sensor according to acquired step counting data of a preset number of steps; and performing step counting according to the calibrated step counting frequency and/or the calibrated effective value of the step counting pace of the three-axis acceleration sensor obtained from the calibration. In the method according to the embodiment of the present invention, a step counting frequency and/or an effective value of a step counting pace of a three-axis acceleration sensor is calibrated. It can avoid the error in the step counting caused by different walking paces of users and the different step counting frequencies of three-axis acceleration sensors. It can improve the accuracy of the step counting data. In addition, through the calibration of the effective step counting frequency, it can process ineffective step counting in the step counting data and further improve the accuracy of the step counting data.

Other aspects will become apparent upon reading and understanding the drawings and detailed description.

### BRIEF DESCRIPTION OF THE DRAWINGS

Fig. 1 is a flowchart of a method for realizing step counting according to an embodiment of the present invention;
Fig. 2 is a block diagram of an apparatus for realizing step counting according to an embodiment of the present invention;
Fig. 3 is schematic diagram of complete displaying a part of unread messages; and
Fig. 4 is a block diagram of an apparatus for displaying an unread message according to an embodiment of the present invention.

### DETAILED DESCRIPTION

Hereinafter, the embodiments of the present invention will be described in detail with reference to the accompanying drawings. It should be noted that, the embodiments in the present application and the features in the embodiments may be arbitrarily combined with each other without conflict.

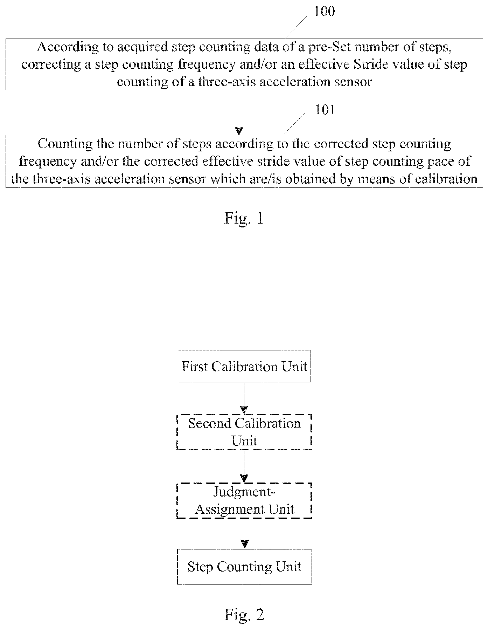

Fig. 1 is a flowchart of a method for realizing step counting according to an embodiment of the present invention. As shown in Fig. 1, the method includes the following steps.

In step 100, a step counting frequency and/or an effective value of a step counting pace of a three-axis acceleration sensor is calibrated according to acquired step counting data of a preset number of steps, the preset number of steps generally refers to about 500 to 1000 steps.

In this step, calibrating the step counting frequency of the three-axis acceleration sensor includes the following steps.

When a step counting value in the step counting data is larger than the actual number of walking steps, the step counting frequency of the three-axis acceleration sensor is reduced by a preset step length within a preset frequency range starting from a first frequency, and a step counting frequency within the preset frequency range when an error between the step counting value and the actual number of walking steps is the smallest, is determined as the calibrated step counting frequency.

When a step counting value in the step counting data is smaller than the actual number of walking steps, the step counting frequency of the three-axis acceleration sensor is increased by a preset step length within a preset frequency range starting from a first frequency, and a step counting frequency within the preset frequency range when an error between the step counting value and the actual number of walking steps is the smallest, is determined as the calibrated step counting frequency.

It should be noted that, the preset frequency range is a general working range determined by a person skilled in the art based on different three-axis acceleration sensors, and the general preset frequency range may be from 8.0Hz to 12.0Hz. The first frequency may be an experience value of a person skilled in the art, for example, 10.OHz. The preset step length may be 0.5Hz.

Calibrating the effective value of the step counting pace includes the following steps.

When a step counting value in the step counting data is larger than the actual number of walking steps, the value of the step counting pace is increased by a preset pace adjusting unit within an effective pace range starting from a first pace, and a value of the step counting pace within the effective pace range when an error between the step counting value and the actual number of walking steps is the smallest, is determined as the calibrated effective value of the step counting pace.

When a step counting value in the step counting data is smaller than the actual number of walking steps, the value of the step counting pace is decreased by a preset pace adjusting unit within an effective pace range starting from a first pace, and a value of the step counting pace within the effective pace range when an error between the step counting value and the actual number of walking steps is the smallest, is determined as the calibrated effective value of the step counting pace.

It should be noted that, the effective pace range is a value range determined by a person skilled in the art according to the pace of a general user, and the general effective pace range may be 5.0cm to 10.0cm. The first pace may be an experience value of a person skilled in the art, for example, 7.5cm. The preset pace adjusting unit may be 0.5cm.

In step 101, the step counting is performed according to the calibrated step counting frequency and/or the calibrated effective value of the step counting pace of the three-axis acceleration sensor obtained from the calibration.

Optionally, the method in the embodiment of the present invention further includes: calibrating an effective step counting frequency to obtain a calibrated effective step counting frequency, to process ineffective step counting in the step counting data.

Optionally, calibrating an effective step counting frequency includes:
When a step counting value in the step counting data is larger than the actual number of walking steps, the effective frequency is reduced by a preset frequency adjusting unit within an effective step counting frequency range starting from a first effective frequency, and an effective frequency within the effective step counting frequency range when an error between the step counting value and the actual number of walking steps is the smallest, is determined as the calibrated effective step counting frequency.

When a step counting value in the step counting data is smaller than the actual number of walking steps, the effective frequency is increased by a preset frequency adjusting unit within an effective step counting frequency range starting from a first effective frequency, and an effective frequency within the effective step counting frequency range when an error between the step counting value and the actual number of walking steps is the smallest, is determined as the calibrated effective step counting frequency.

It should be noted that the effective step counting frequency range is a value range determined by a person skilled in the art according to the frequency of the walking of a general user, and the walking speed of a general user does not exceed 3 steps per second. According to the analysis, the time for each step of the user is 200 milliseconds to 1000 milliseconds, and generally, the time for walking one step is 300 milliseconds. In the calibration, 50 milliseconds may be taken as a time period for adjusting, and a corresponding frequency may be calculated according to the above time period.

In the method of the embodiment of the present invention, if the calibration process includes calibrating the step counting frequency, the effective value of the step counting pace and the effective step counting frequency of the three-axis acceleration sensor, the calibration process includes the following steps.

After the calibrated step counting frequency is obtained, the effective value of the step counting pace is calibrated according to the step counting data based on the calibrated step counting frequency, and then, the effective step counting frequency is calibrated according to the step counting data based on the calibrated effective value of the step counting pace.

The method of the embodiment of the present invention further includes: when the calibration of the step counting frequency does not reduce the error between the step counting value and the actual number of walking steps, taking the first frequency as the calibrated step counting frequency;
when the calibration of the effective value of the step counting pace does not reduce the error between the step counting value and the actual number of walking steps, taking the first pace as the calibrated effective value of the step counting pace; and
when the calibration of the effective step counting frequency does not reduce the error between the step counting value and the actual number of walking steps, taking the first effective frequency as the calibrated effective step counting frequency.

The method for calibrating the step counting frequency according to the embodiment of the present invention further includes:

based on machine learning algorithm and migration learning algorithm, taking accelerometer data of the three-axis acceleration sensor of the actual number of walking steps in the step counting data, the first frequency and the step counting value as input, a step counting frequency when an error between the step counting value and the actual number of walking steps is the smallest during the calculation within the preset frequency range with the preset step length, is determined as the calibrated step counting frequency.

Calibrating the effective value of the step counting pace includes:
based on machine learning algorithm and migration learning algorithm, taking accelerometer data of the three-axis acceleration sensor of the actual number of walking steps in the step counting data, the first pace and the step counting value as input, a value of the step counting pace when an error between the step counting value and the actual number of walking steps is the smallest during the calculation within the effective pace range with the preset pace adjusting unit, is determined as the calibrated effective value of the step counting pace.

Calibrating the effective step counting frequency includes:
based on machine learning algorithm and migration learning algorithm, taking accelerometer data of the three-axis acceleration sensor of the actual number of walking steps in the step counting data, the first effective frequency and the step counting value as input, an effective frequency value when an error between the step counting value and the actual number of walking steps is the smallest within the effective step counting frequency range with the preset frequency adjusting unit, is determined as the calibrated effective step counting frequency.

It should be noted that the machine learning algorithm and the migration learning algorithm may be a decision tree algorithm, a neural network algorithm, an extreme learning machine algorithm or a deep learning algorithm, and so on.

According to the method of the embodiment of the present invention, a step counting frequency and/or an effective value of a step counting pace of a three-axis acceleration sensor is calibrated, to avoid the error in the step counting caused by different walking paces of users and the different step counting frequencies of three-axis acceleration sensors. It can improve the accuracy of the step counting data. In addition, through the calibration of the effective step counting frequency, it can process ineffective step counting in the step counting data and further improve the accuracy of the step counting data.

An embodiment of the present invention further provides a computer storage medium for storing computer-executable instructions which implement the above method.

Fig. 2 is a block diagram of an apparatus for realizing step counting according to an embodiment of the present invention. As shown in Fig. 2, the apparatus includes a first calibration unit and a step counting unit.

The first calibration unit is configured to calibrate a step counting frequency and/or an effective value of a step counting pace of a three-axis acceleration sensor according to acquired step counting data of a preset number of steps.

The first calibration unit is configured to acquire step counting data of a preset number of steps;
when a step counting value in the step counting data is larger than the actual number of walking steps, reduce the step counting frequency of the three-axis acceleration sensor by a preset step length within a preset frequency range starting from a first frequency, and determine a step counting frequency within the preset frequency range when an error between the step counting value and the actual number of walking steps is the smallest, as the calibrated step counting frequency;
when a step counting value in the step counting data is smaller than the actual number of walking steps, increase the step counting frequency of the three-axis acceleration sensor by a preset step length within a preset frequency range starting from a first frequency, and determine a step counting frequency within the preset frequency range when an error between the step counting value and the actual number of walking steps is the smallest, as the calibrated step counting frequency; and/or
when a step counting value in the step counting data is larger than the actual number of walking steps, increase the value of the step counting pace by a preset pace adjusting unit within an effective pace range starting from a first pace, and determine a value of the step counting pace within the effective pace range when an error between the step counting value and the actual number of walking steps is the smallest, as the calibrated effective value of the step counting pace;

when a step counting value in the step counting data is smaller than the actual number of walking steps, decrease the value of the step counting pace by a preset pace adjusting unit within an effective pace range starting from a first pace, and determine a value of the step counting pace within the effective pace range when an error between the step counting value and the actual number of walking steps is the smallest, as the calibrated effective value of the step counting pace.

The step counting unit is configured to perform the step counting according to the calibrated step counting frequency and/or the calibrated effective value of the step counting pace of the three-axis acceleration sensor obtained from the calibration.

The apparatus according to the embodiment of present invention also includes a second calibration unit, configured to calibrate an effective step counting frequency to obtain a calibrated effective step counting frequency, to process ineffective step counting in the step counting data from the step counting unit.

The second calibration unit is configured to acquire step counting data of a preset number of steps;
when a step counting value in the step counting data is larger than the actual number of walking steps, reduce the effective frequency by a preset frequency adjusting unit within an effective step counting frequency range starting from a first effective frequency, and determine an effective frequency within the effective step counting frequency range when an error between the step counting value and the actual number of walking steps is the smallest, as the calibrated effective step counting frequency; and
when a step counting value in the step counting data is smaller than the actual number of walking steps, increase the effective frequency by a preset frequency adjusting unit within an effective step counting frequency range starting from a first effective frequency, and determine an effective frequency within the effective step counting frequency range when an error between the step counting value and the actual number of walking steps is the smallest, as the calibrated effective step counting frequency.

The first calibration unit is configured to, after the calibrated step counting frequency of the three-axis acceleration sensor is obtained according to the acquired step counting data of a preset number of steps, calibrate the effective value of the step counting pace according to the step counting data based on the calibrated step counting frequency.

The second calibration unit is configured to, after the first calibration unit has completed the calibration of the step counting frequency of the three-axis acceleration sensor and then the calibration of the effective value of the step counting pace sequentially, calibrate the effective step counting frequency according to the step counting data based on the calibrated effective value of the step counting pace.

The apparatus according to the embodiment of the present invention also includes a judgment-assignment unit, configured to,
when the calibration of the step counting frequency does not reduce the error between the step counting value and the actual number of walking steps, take the first frequency as the calibrated step counting frequency;
when the calibration of the effective value of the step counting pace does not reduce the error between the step counting value and the actual number of walking steps, take the first pace as the calibrated effective value of the step counting pace; and
when the calibration of the effective step counting frequency does not reduce the error between the step counting value and the actual number of walking steps, take the first effective frequency as the calibrated effective step counting frequency.

The first calibration unit is configured to, based on machine learning algorithm and migration learning algorithm, taking accelerometer data of the three-axis acceleration sensor of the actual number of walking steps in the step counting data, the first frequency and the step counting value as input, determine a step counting frequency when an error between the step counting value and the actual number of walking steps is the smallest during the calculation within the preset frequency range with the preset step length, as the calibrated step counting frequency; and/or

based on machine learning algorithm and migration learning algorithm, taking accelerometer data of the three-axis acceleration sensor of the actual number of walking steps in the step counting data, the first pace and the step counting value as input, determine a value of the step counting pace when an error between the step counting value and the actual number of walking steps is the smallest during the calculation within the effective pace range with the preset pace adjusting unit, as the calibrated effective value of the step counting pace.

The second calibration unit is configured to, based on machine learning algorithm and migration learning algorithm, taking accelerometer data of the three-axis acceleration sensor of the actual number of walking steps in the step counting data, the first effective frequency and the step counting value as input, determine an effective frequency when an error between the step counting value and the actual number of walking steps is the smallest within the effective pace range with the preset pace adjusting unit, as the calibrated effective step counting frequency.

It should be noted that, in the embodiment of the present invention, the apparatus for step counting may perform step counting individually, or may be embedded in a mobile terminal, for example, performing step counting in the mobile terminal. The specific embedding mode does not require the skilled person in the art to pay creative effort.

The method of the present invention will be described in detail below through a specific embodiment. The embodiment is only used for illustrating the present invention, and is not intended to limit the protection scope of the method of the present invention.

In this embodiment, the first frequency of the three-axis acceleration sensor is 10.OHz, the preset frequency range may take the value of 8.0Hz to 12.0Hz and the preset step length is 0.5Hz. The effective pace range may be 5.0cm to 10.0cm, the first pace is 7.5cm, the preset pace adjusting unit is 0.5cm. The effective step counting frequency range is determined within 200 milliseconds to 1000 milliseconds according to the time for each step of the user, the first effective frequency is 300 milliseconds for each step for example, and the frequency adjusting unit is 50 milliseconds.

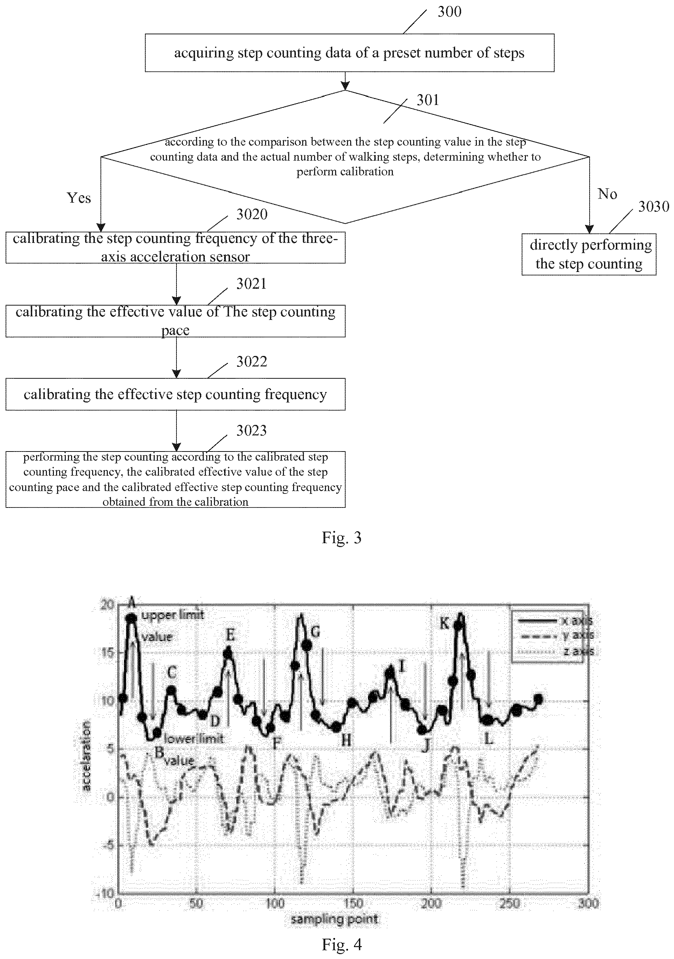

Fig. 3 is a flowchart of a method for performing step counting according to an embodiment of the present invention. As shown in FIG. 3, the method includes the following steps.

In Step 300, step counting data of a preset number of steps and an actual number of walking steps are acquired. Acquiring the actual number of walking steps includes: the three-axis acceleration sensor acquiring x, y, z axes acceleration data and the step counting value, and the like in real time.

In Step 301, according to the comparison between the step counting value in the step counting data and the actual number of walking steps, it is determined whether to perform calibration. Optionally, when the step counting value is larger than or smaller than the actual number of walking steps, it may be determined to perform calibration. It should be noted that, in practical application, an error ratio may be setup for determining whether to perform calibration. For example, when an error between the step counting value and the actual number of walking steps exceeds 2%, calibration is required. The specific percentage may be adjusted according to actual situation. Here, the calibration includes calibration of the step counting frequency, and/or the effective value of the step counting pace, and/or the effective step counting frequency of the three-axis acceleration sensor. In the present embodiment, it is assumed that there is an error between the step counting value and the actual number of walking steps, and calibration is required in Step 3020. If there is no error, or the error ration is smaller than the error ratio setup by the technician, no calibration is needed, and the process directly proceeds to Step 3030 to perform step counting according to relevant parameters.

In Step 3020, the step counting frequency of the three-axis acceleration sensor is calibrated.

Optionally, when the step counting value in the step counting data is larger than the actual number of walking steps, the step counting frequency of the three-axis acceleration sensor is reduced by the step length 0.5Hz within the range 10Hz ∼ 8Hz starting from 10.OHz, and a step counting frequency within the preset frequency range when an error between the step counting value and the actual number of walking steps is the smallest, is determined as the calibrated step counting frequency. Here, preset frequency range is theoretically 8Hz to 12Hz. However, since the first frequency is 10Hz and reduction of the step counting frequency is performed, the adjustment range is reduced to 10Hz ∼ 8Hz from the theoretically 8Hz to 12Hz. This is similar in the following description regarding the calibration, which will not be repeated.

When the step counting value in the step counting data is smaller than the actual number of walking steps, the step counting frequency of the three-axis acceleration sensor is increased by the step length 0.5Hz within the range 10Hz ∼ 12Hz starting from 10.OHz, and a step counting frequency within the preset frequency range when an error between the step counting value and the actual number of walking steps is the smallest, is determined as the calibrated step counting frequency.

When the calibration of the step counting frequency does not reduce the error between the step counting value and the actual number of walking steps, the first frequency is taken as the calibrated step counting frequency.

In Step 3021, the effective step counting frequency is calibrated.

Optionally, when the step counting value in the step counting data is larger than the actual number of walking steps, the value of the step counting pace is increased by a preset pace adjusting unit 0.5cm within 7.5cm∼10.0cm starting from 7.5cm, and a value of the step counting pace within the effective pace range when an error between the step counting value and the actual number of walking steps is the smallest, is determined as the calibrated effective value of the step counting pace.

When the step counting value in the step counting data is smaller than the actual number of walking steps, the value of the step counting pace is decreased by a preset pace adjusting unit 0.5cm within 5cm∼7.5cm starting from 7.5cm, and a value of the step counting pace within the effective pace range when an error between the step counting value and the actual number of walking steps is the smallest, is determined as the calibrated effective value of the step counting pace.

When the calibration of the effective value of the step counting pace does not reduce the error between the step counting value and the actual number of walking steps, the first pace is taken as the calibrated effective value of the step counting pace.

Fig. 4 is a graph of sampled three-axis acceleration during the step counting process according to the embodiment. As shown in Fig. 4, the horizontal axis represents the sampling points, the vertical axis represents the acceleration values, the solid line represents the x-axis acceleration data, the dotted line represents the z-axis acceleration data, and the dashed line represents the y-axis acceleration data. In Fig. 4, the x-axis acceleration data fluctuated more significantly, which may be determined as acceleration in the gravitational direction. In Fig. 4, at the point A, the changing direction is changed from upward to downward, therefore, the point A is an upper limit value point. At the point B, the changing direction is changed from downward to upward, therefore, the point B is a lower limit value point. Similarly, the subsequent points C, E, G, I and K are upper limit value points, and points D, F, H, J and L are lower limit value points. The pace value between an upper limit value point and a lower limit value point is a pace of a step. When the pace satisfies the length of the effective value of the step counting pace, the step counting may be performed. In the present embodiment, before the calibration, the first pace is 7.5cm. In Fig. 4, the pace value between the point A and the point B is 12cm, which is larger than 7.5cm; the pace value between the point B and the point C is 4.5cm, which is smaller than 7.5cm; the pace value between the point C and the point D is 3cm, which is smaller than 7.5cm, and so on. It may be obtained from calculation that the step counting value is 3 based on the first pace 7.5cm. It is assumed that the step counting value is smaller than the actual number of walking steps. Then, the value of the step counting pace is reduced at the pace adjusting unit 0.5cm, that is, the value of the step counting pace is reduced sequentially to 7cm, 6.5cm, 6cm. It is assumed that when the value of the step counting pace is reduced to 6cm, the step counting value is 5, which is accurate step counting data. Then, 6cm is determined as the calibrated effective value of the step counting pace.

In Step 3022, effective step counting frequency is calibrated.

Optionally, when the step counting value in the step counting data is larger than the actual number of walking steps, the effective frequency is reduced by the preset frequency adjusting unit within the effective step counting frequency range starting from the first effective frequency, and an effective frequency within the effective step counting frequency range when an error between the step counting value and the actual number of walking steps is the smallest, is determined as the calibrated effective step counting frequency.

When the step counting value in the step counting data is smaller than the actual number of walking steps, the effective frequency is increased by the preset frequency adjusting unit within the effective step counting frequency range starting from the first effective frequency, and an effective frequency within the effective step counting frequency range when an error between the step counting value and the actual number of walking steps is the smallest, is determined as the calibrated effective step counting frequency.

When the calibration of the effective step counting frequency does not reduce the error between the step counting value and the actual number of walking steps, the first effective frequency is taken as the calibrated effective step counting frequency.

In Step 3023, the step counting is performed according to the calibrated step counting frequency, the calibrated effective value of the step counting pace and the calibrated effective step counting frequency obtained from the calibration.

In Step 3023, the step counting is directly performed.

In this embodiment, the step counting frequency of the three-axis acceleration sensor is calibrated, to avoid the situation that when the step counting frequency is too high, the step counting value is too large, and when the step counting frequency is too low, the step counting value is too small. The effective value of the step counting pace is calibrated, to avoid the situation that the space data is influenced due to that different users have different paces, walking postures, walking habits and different positions to put the step counting apparatus. The effective step counting frequency is calibrated, to regard the step counting not caused by walking as ineffective step counting. For example, a user puts a mobile phone for step counting in a bag or holds it in hand. When the user walks, since the mobile phone slides in the bag or in the hand, the three-axis acceleration sensor is initially influenced and performs step counting, and generates data outside the effective step counting frequency range. This part of data is processed, which can improve the accuracy of step counting. According to experiments, through the method of the present embodiment, the error in step counting can be reduced from more than 8% to about 2%. Therefore, the accuracy of step counting can be improved.

In this embodiment, the calibration process may be implemented by a machine learning algorithm and a migration learning algorithm, and specifically may be a decision tree algorithm, a neural network algorithm, an extreme learning machine algorithm, a deep learning algorithm, or the like.

A person of ordinary skill in the art may understand that all or part of the steps in the foregoing methods may be implemented by a program instructing relevant hardware (for example, a processor), and the program may be stored in a computer-readable storage medium, such as a read-only memory, a magnetic disk, or a compact disc, etc. Optionally, all or part of the steps of the above embodiments may also be implemented by using one or more integrated circuits. Correspondingly, each module / unit in the above embodiments can be implemented in the form of hardware. For example, the integrated circuit can realize its corresponding function, and can also be implemented in the form of software function module, for example, the processor executes the program / instructions stored in the memory to achieve its corresponding function. The present invention is not limited to any particular form of combination of hardware and software.

Any modification and variation may be made by those skilled in the art without departing from the spirit and scope of the present invention, but the scope of protection of the present invention is subject to the scope defined by the appended claims.

### Industrial Applicability

The above technical solutions can avoid the error in the step counting caused by different walking paces of users and the different step counting frequencies of three-axis acceleration sensors. It can improve the accuracy of the step counting data. In addition, through the calibration of the effective step counting frequency, it can process ineffective step counting in the step counting data and further improve the accuracy of the step counting data.

## Claims

1. A method for realizing step counting, comprising:
calibrating a step counting frequency and/or an effective value of a step counting pace of a three-axis acceleration sensor according to acquired step counting data of a preset number of steps; and
performing step counting according to the calibrated step counting frequency and/or the calibrated effective value of the step counting pace of the three-axis acceleration sensor obtained from the calibration.

2. The method according to claim 1, further comprising:
calibrating an effective step counting frequency to obtain a calibrated effective step counting frequency; and
processing ineffective step counting in the step counting data according to the calibrated effective step counting frequency.

3. The method according to claim 2, wherein calibrating the step counting frequency of the three-axis acceleration sensor comprises:
when a step counting value in the step counting data is larger than an actual number of walking steps, reducing the step counting frequency of the three-axis acceleration sensor by a preset step length within a preset frequency range starting from a first frequency, and determining a step counting frequency within the preset frequency range when an error between the step counting value and the actual number of walking steps is the smallest, as the calibrated step counting frequency; and
when the step counting value in the step counting data is smaller than the actual number of walking steps, increasing the step counting frequency of the three-axis acceleration sensor by a preset step length within a preset frequency range starting from a first frequency, and determining a step counting frequency within the preset frequency range when an error between the step counting value and the actual number of walking steps is the smallest, as the calibrated step counting frequency.

4. The method according to claim 2, wherein calibrating the effective value of the step counting pace comprises:
when a step counting value in the step counting data is larger than an actual number of walking steps, increasing the value of the step counting pace by a preset pace adjusting unit within an effective pace range starting from a first pace, and determining a value of the step counting pace within the effective pace range when an error between the step counting value and the actual number of walking steps is the smallest, as the calibrated effective value of the step counting pace; and
when a step counting value in the step counting data is smaller than the actual number of walking steps, decreasing the value of the step counting pace by a preset pace adjusting unit within an effective pace range starting from a first pace, and determining a value of the step counting pace within the effective pace range when an error between the step counting value and the actual number of walking steps is the smallest, as the calibrated effective value of the step counting pace.

5. The method according to claim 3 or 4, wherein calibrating an effective step counting frequency comprises:
when a step counting value in the step counting data is larger than the actual number of walking steps, reducing the effective frequency by a preset frequency adjusting unit within an effective step counting frequency range starting from a first effective frequency, and determining an effective frequency within the effective step counting frequency range when an error between the step counting value and the actual number of walking steps is the smallest, as the calibrated effective step counting frequency; and
when a step counting value in the step counting data is smaller than the actual number of walking steps, increasing the effective frequency by a preset frequency adjusting unit within an effective step counting frequency range starting from a first effective frequency, and determining an effective frequency within the effective step counting frequency range when an error between the step counting value and the actual number of walking steps is the smallest, as the calibrated effective step counting frequency.

6. The method according to claim 2, wherein calibrating a step counting frequency and an effective value of a step counting pace of a three-axis acceleration sensor comprises:
after the calibrated step counting frequency is obtained, calibrating the effective value of the step counting pace according to the step counting data based on the calibrated step counting frequency; and
calibrating a step counting frequency, an effective value of a step counting pace and an effective step counting frequency of a three-axis acceleration sensor comprises:
after the calibrated step counting frequency is obtained, calibrating a parameter of the effective value of the step counting pace according to the step counting data based on the calibrated step counting frequency; and calibrating the effective step counting frequency according to the step counting data based on the calibrated effective value of the step counting pace.

7. The method according to claim 5, further comprising:
when the calibration of the step counting frequency does not reduce the error between the step counting value and the actual number of walking steps, taking the first frequency as the calibrated step counting frequency;
when the calibration of the effective value of the step counting pace does not reduce the error between the step counting value and the actual number of walking steps, taking the first pace as the calibrated effective value of the step counting pace; and
when the calibration of the effective step counting frequency does not reduce the error between the step counting value and the actual number of walking steps, taking the first effective frequency as the calibrated effective step counting frequency.

8. The method according to claim 2, wherein,
calibrating the step counting frequency comprises:
based on machine learning algorithm and migration learning algorithm, taking accelerometer data of the three-axis acceleration sensor of the actual number of walking steps in the step counting data, the first frequency and the step counting value as input, determining a step counting frequency when an error between the step counting value and the actual number of walking steps is the smallest during the calculation within the preset frequency range with the preset step length, as the calibrated step counting frequency;
calibrating the effective value of the step counting pace comprises:
based on machine learning algorithm and migration learning algorithm, taking accelerometer data of the three-axis acceleration sensor of the actual number of walking steps in the step counting data, the first pace and the step counting value as input, determining a value of the step counting pace when an error between the step counting value and the actual number of walking steps is the smallest during the calculation within the effective pace range with the preset pace adjusting unit, as the calibrated effective value of the step counting pace; and
calibrating the effective step counting frequency comprises:
based on machine learning algorithm and migration learning algorithm, taking accelerometer data of the three-axis acceleration sensor of the actual number of walking steps in the step counting data, the first effective frequency and the step counting value as input, determining an effective frequency when an error between the step counting value and the actual number of walking steps is the smallest within the effective pace range with the preset pace adjusting unit, as the calibrated effective step counting frequency.

9. An apparatus for realizing step counting, comprising:
a first calibration unit configured to calibrate a step counting frequency and/or an effective value of a step counting pace of a three-axis acceleration sensor according to acquired step counting data of a preset number of steps; and
a step counting unit configured to perform step counting according to the calibrated step counting frequency and/or the calibrated effective value of the step counting pace of the three-axis acceleration sensor obtained from the calibration.

10. The apparatus according to claim 9, further comprising:
a second calibration unit configured to calibrate an effective step counting frequency to obtain a calibrated effective step counting frequency; and process ineffective step counting in the step counting data from the step counting unit according to the calibrated effective step counting frequency.

11. The apparatus according to claim 10, wherein
the first calibration unit is configured to calibrate the step counting frequency of the three-axis acceleration sensor by:
according to acquired step counting data of a preset number of steps,
when a step counting value in the step counting data is larger than an actual number of walking steps, reducing the step counting frequency of the three-axis acceleration sensor by a preset step length within a preset frequency range starting from a first frequency, and determining a step counting frequency within the preset frequency range when an error between the step counting value and the actual number of walking steps is the smallest, as the calibrated step counting frequency; and
when the step counting value in the step counting data is smaller than the actual number of walking steps, increasing the step counting frequency of the three-axis acceleration sensor by a preset step length within a preset frequency range starting from a first frequency, and determining a step counting frequency within the preset frequency range when an error between the step counting value and the actual number of walking steps is the smallest, as the calibrated step counting frequency;
the first calibration unit is configured to calibrate the effective value of the step counting pace by:
when a step counting value in the step counting data is larger than an actual number of walking steps, increasing the value of the step counting pace by a preset pace adjusting unit within an effective pace range starting from a first pace, and determining a value of the step counting pace within the effective pace range when an error between the step counting value and the actual number of walking steps is the smallest, as the calibrated effective value of the step counting pace; and
when a step counting value in the step counting data is smaller than the actual number of walking steps, decreasing the value of the step counting pace by a preset pace adjusting unit within an effective pace range starting from a first pace, and determining a value of the step counting pace within the effective pace range when an error between the step counting value and the actual number of walking steps is the smallest, as the calibrated effective value of the step counting pace.

12. The apparatus according to claim 11, wherein the second calibration unit is configured to calibrate an effective step counting frequency by:
according to acquired step counting data of a preset number of steps,
when a step counting value in the step counting data is larger than the actual number of walking steps, reducing the effective frequency by a preset frequency adjusting unit within an effective step counting frequency range starting from a first effective frequency, and determining an effective frequency within the effective step counting frequency range when an error between the step counting value and the actual number of walking steps is the smallest, as the calibrated effective step counting frequency; and
when a step counting value in the step counting data is smaller than the actual number of walking steps, increasing the effective frequency by a preset frequency adjusting unit within an effective step counting frequency range starting from a first effective frequency, and determining an effective frequency within the effective step counting frequency range when an error between the step counting value and the actual number of walking steps is the smallest, as the calibrated effective step counting frequency.

13. The apparatus according to claim 12, wherein the first calibration unit is configured to calibrate a step counting frequency and an effective value of a step counting pace of a three-axis acceleration sensor by:
after calibrating the step counting frequency of the three-axis acceleration sensor to obtain the calibrated step counting frequency according to the acquired step counting data of a preset number of steps, calibrating the effective value of the step counting pace according to the step counting data based on the calibrated step counting frequency; and
the second calibration unit is configured to calibrate a step counting frequency, an effective value of a step counting pace and an effective step counting frequency of a three-axis acceleration sensor by:
after the first calibration unit has completed the calibration of the step counting frequency of the three-axis acceleration sensor and then the calibration of the effective value of the step counting pace sequentially, calibrating the effective step counting frequency according to the step counting data based on the calibrated effective value of the step counting pace.

14. The apparatus according to claim 12 or 13, further comprising:
a judgment-assignment unit configured to, when the calibration of the step counting frequency does not reduce the error between the step counting value and the actual number of walking steps, take the first frequency as the calibrated step counting frequency;
when the calibration of the effective value of the step counting pace does not reduce the error between the step counting value and the actual number of walking steps, take the first pace as the calibrated effective value of the step counting pace; and
when the calibration of the effective step counting frequency does not reduce the error between the step counting value and the actual number of walking steps, take the first effective frequency as the calibrated effective step counting frequency.

15. The apparatus according to claim 10, wherein,
the first calibration unit is configured to calibrate the step counting frequency by:
based on machine learning algorithm and migration learning algorithm, taking accelerometer data of the three-axis acceleration sensor of the actual number of walking steps in the step counting data, the first frequency and the step counting value as input, determining a step counting frequency when an error between the step counting value and the actual number of walking steps is the smallest during the calculation within the preset frequency range with the preset step length, as the calibrated step counting frequency;
the first calibration unit is configured to calibrate the effective value of the step counting pace by:
based on machine learning algorithm and migration learning algorithm, taking accelerometer data of the three-axis acceleration sensor of the actual number of walking steps in the step counting data, the first pace and the step counting value as input, determining a value of the step counting pace when an error between the step counting value and the actual number of walking steps is the smallest during the calculation within the effective pace range with the preset pace adjusting unit, as the calibrated effective value of the step counting pace; and
the second calibration unit is configured to calibrate the effective step counting frequency comprises:
based on machine learning algorithm and migration learning algorithm, taking accelerometer data of the three-axis acceleration sensor of the actual number of walking steps in the step counting data, the first effective frequency and the step counting value as input, determining an effective frequency when an error between the step counting value and the actual number of walking steps is the smallest within the effective frequency range with the preset frequency adjusting unit, as the calibrated effective step counting frequency.

16. A computer storage medium having stored therein computer-executable instructions for performing the method of any one of claims 1-8.
